# FASCICULE DE BREVET EUROPEEN

(11) **EP 3 403 096 B1**
(45) Date de publication et mention de la délivrance du brevet: **08.03.2023**
(21) Numéro de dépôt: 17712189.4
(22) Date de dépôt: 12.01.2017
(51) Int. Cl.: G01N 33/543, G01N 33/569

(54) **PROCÉDÉ DE DÉTERMINATION D'UNE RÉPONSE HUMORALE CHEZ UN SUJET IMMUNODÉPRIMÉ**
VERFAHREN ZUR ERMITTLUNG EINER HUMORALEN ANTWORT BEI EINEM IMMUNSUPPRIMIERTEN PATIENTEN
METHOD FOR DETERMINING HUMORAL RESPONSE IN AN IMMUNOSUPRESSED PATIENT

(30) Priorité: 14.01.2016 FR 1650280
(43) Date de publication de la demande: 21.11.2018
(73) Titulaire: bioMérieux, 69280 Marcy-l'Étoile (FR)
(72) Inventeur: BOUCHARD, Ghislaine, 69003 Lyon (FR)
(74) Mandataire: Cabinet Beau de Loménie
(86) Numéro de dépôt international: PCT/FR2017/050068
(87) Numéro de publication internationale: WO 2017/121963

(56) Documents cités:
- CN-A- 104 330 572
- ZHANG S.X.: "Non-culture-based methods in diagnostic mycology", CLIN. MICROBIOL. NEWSLETT., vol. 34, no. 13, 1 juillet 2012 (2012-07-01), pages 101-105, XP028495139,
- MACEDO DE OLIVEIRA A. ET AL.: "Sensitivity of second-generation enzyme immunoassay for detection of hepatitis C virus infection among oncology patients", J. CLIN. VIROL., vol. 35, no. 1, janvier 2006 (2006-01), pages 21-25, XP028038053,

## Description

La présente invention concerne le domaine des procédés de détection de la réponse humorale chez un sujet, en réponse à un agent infectieux. L'invention est destinée à des sujets immunodéprimés.

En réponse à une substance étrangère, en particulier à un agent infectieux (bactéries, virus, champignons et parasites), l'organisme d'un sujet déclenche une réponse immunitaire. La réponse humorale correspond à la production d'immunoglobulines (anticorps). Ces immunoglobulines se diffusent dans le sang, les tissus ou les muqueuses du sujet et vont permettre à l'organisme de se défendre contre l'agent infectieux. Une telle défense peut se matérialiser par une capacité à neutraliser ou à empêcher la reproduction de l'agent infectieux ou une nouvelle infection par l'agent infectieux

Actuellement, une fraction non négligeable de la population est en situation d'immunodépression. Cette immunodépression est la conséquence de diverses situations cliniques regroupant notamment les déficits immunitaires congénitaux, les déficits dus au vieillissement du système immunitaire (immunosénescence) que l'on observe à partir d'un âge supérieur à 65 ans, les déficits reliés à diverses pathologies (diabète, réaction inflammatoire chronique, néoplasie, hémopathie, hépatopathie chronique, infection par VIH, insuffisance rénale chronique... ), les déficits immunitaires induits par certains traitements médicaux (corticothérapie, interféron, traitement immunosuppresseur accompagnant les acceptations de greffon ...) ou actes chirurgicaux (splénectomie).

Le patient immunodéprimé présente une plus grande susceptibilité aux agents infectieux viraux, bactériens et/ou fongiques, et de ce fait nécessite une prise en charge médicale adaptée tant pour les approches et les outils diagnostiques que pour les traitements prophylactiques ou thérapeutiques. Chaque situation d'immunodépression est complexe et se caractérise par un profil immunologique spécifique.

Les tests de diagnostic *in vitro* (IVD) sont un des outils utilisés pour la détection des immunoglobulines (anticorps) spécifiques d'un agent infectieux et pour déterminer la réponse humorale d'un sujet vis-à-vis dudit agent infectieux. Un test IVD rend un résultat de détection négative (absence de réponse humorale correspondant à un résultat considéré comme une absence d'anticorps) ou positive (présence de réponse humorale correspondant à un résultat considéré comme la présence d'anticorps), séparant les résultats négatifs (spécificité du test) et positifs (sensibilité du test) par un seuil de positivité ou signal de référence.

En amont d'une immunosuppression programmée (greffe, splénectomie, par exemple), il est possible d'assurer la protection du patient par certaines vaccinations anti-infectieuses. La qualité de la protection vaccinale est assurée par la présence d'immunoglobulines spécifiques pouvant être évaluée par le résultat du test IVD correspondant.

Cependant, il est connu que l'immunosuppression peut être responsable de la régression, voire de la disparition, de la protection immunitaire vaccinale (Haut Conseil de la Santé Publique, Vaccination des personnes immunodéprimées ou aspléniques ; Recommandations ; Rapport 2012).

Un suivi de la régression peut être effectué par les résultats successifs de tests IVD. Dans ce suivi spécifique, le test IVD peut être pris en défaut lorsque la situation du patient évolue, faisant sortir ses résultats des valeurs usuellement reconnues pour la population normale.

En effet, un test IVD annonce des performances de sensibilité et spécificité validées sur des cohortes d'échantillons, jugés représentatifs de la population normale.

Les sérums, ou plus généralement les échantillons, de patients immunodéprimés répondent différemment dans les tests de diagnostic ciblant un agent infectieux. En effet, l'immunosuppression peut être responsable d'une diminution plus ou moins accentuée du taux d'immunoglobulines et ce déficit en immunoglobulines peut être à l'origine d'un défaut de réponse dans le test. La population immunodéprimée n'est pas représentative de la population normale et le seuil de positivité du test, déterminé avec des échantillons de population normale, n'est plus pertinent (voir notamment Zhang et al., Clin. Microbiol. Newslett. 2012, 34, 13, 101-105 et Macedo de Oliveira et al., J. Clin. Virol. 2006, 35, 21-25).

Le développement de tests spécifiques pour la population immunodéprimée est difficile car l'immunosuppression est statistiquement non normale, correspond à une situation individuelle, d'intensité quantitativement et qualitativement variable, et, de surcroit transitoire si elle est induite par un traitement immunosuppresseur ponctuel ou une pathologie non chronique. Cependant, les patients immunodéprimés, plus sensibles à l'infection, ont besoin de tests de diagnostic spécifiques dans lesquels le seuil de positivité est également représentatif, malgré la spécificité de la situation.

Le problème technique que se propose de résoudre l'invention est de permettre à l'homme du métier d'utiliser une trousse ou un dispositif de détection de la réponse humorale, initialement destiné à une population non immunodéprimée, sur une population immunodéprimée, sans changer les réactifs de détection de la trousse ou dispositif.

Dans ce contexte, l'invention concerne un procédé de détermination chez un sujet immunodéprimé d'une réponse humorale due à la présence d'un agent infectieux cible, par détection dans un échantillon biologique **E1** dudit sujet, d'au moins un anticorps cible susceptible d'être produit par ledit sujet, ou plus précisément par l'organisme dudit sujet, lorsque ce dernier est infecté ou a été infecté par ledit agent infectieux cible, comprenant les étapes suivantes :
a) disposer d'un volume **V1** de l'échantillon **E1,**
b) diluer le volume **V1,** avec un volume **D1** de diluant, selon le rapport de dilution **R1** qui est égal à **V1/(V1+D1),** avec le rapport de dilution **R1** étant supérieur au rapport de dilution **R2** et **1,5R2 ≤ R1 ≤ 5R2, R2** étant le rapport de dilution utilisé dans le cas d'un sujet non immunodéprimé et **R2** étant égal à **V2/(V2+D2)** avec **V2** et **D2** qui sont respectivement le volume d'échantillon **E2** et le volume de diluant utilisés dans le cas d'un sujet non immunodéprimé, de manière à obtenir un échantillon dilué,
c) réaliser la détection dudit au moins anticorps cible sur l'échantillon dilué obtenu, de manière à conclure quant à la présence éventuelle de la réponse humorale.

L'invention propose d'utiliser une même trousse ou un même dispositif de détection *in vitro* d'anticorps, validé pour un usage sur une population normale, pour rendre un résultat fiable également chez des patients en situation d'immunodépression.

La description détaillée qui va suivre va permettre de mieux comprendre l'invention.

Le procédé selon l'invention utilise une trousse ou dispositif adapté à la détection d'anticorps et donc à la détection d'une réponse humorale chez un sujet. Par « trousse », on entend un ensemble de réactifs nécessaires à la détection, devant être mis en oeuvre avec un instrument intégrant ou non des composantes électroniques. Par « dispositif », on entend un ensemble intégrant à la fois les réactifs nécessaires à la détection et l'instrument pour leur mise en oeuvre.

Dans le cadre de l'invention, la réponse humorale détectée est due à la présence actuelle ou passé de l'agent infectieux entraînant une telle réponse. Par « agent infectieux », on entend un agent biologique pathogène, responsable d'une infection ou infestation, et donc les virus, les bactéries, les parasites et les champignons, en particulier, les champignons inférieurs (ou microscopiques).

Les bactéries pourront être aérobies ou anaérobies. On peut citer, en particulier, les bactéries *Clostridium difficile, Helicobacter pylori, Mycoplasma pneumoniae, Treponema pallidum* (responsable de la Syphilis) et *Borrelia burgdorferi sensu lato* (responsable de la maladie de Lyme).

A titre d'exemple de virus, on peut citer le virus responsable de la rubéole, le cytomegalovirus (CMV), les virus de l'herpès (HHV-1 à HHV-8), le virus responsable de la rougeole, le virus responsable des oreillons, le virus responsable de la varicelle, les virus hépatiques : HBV, HCV, HAV et HEV, le virus HIV, le virus Epstein-Barr EBV (de la mononucléose), le virus du Nil occidental, le virus de la dengue et les papillomavirus et en particulier HPV-16 et HPV-18.

Les champignons peuvent être unicellulaires (levures) ou pluricellulaires (champignons filamenteux ou moisissures). A titre d'exemple de champignons, on peut citer les *Candida* et en particulier *Candida albicans,* les *Cryptococcus* et en particulier *Cryptococcus neoformans,* et les *Aspergillus* et en particulier *A. fumigatus, A. niger, A. nidulans, A. fiavus, A. davatus.*

Les parasites, organismes de taille variable qui vivent aux dépens d'un autre organisme, peuvent être des ectoparasites ou des endoparasites, plus précisément, des arthropodes ou des helminthes tels que les oxyures, le Ténia, les Ascaris qui engendrent les ascaridioses, le Schistosome qui engendre la bilharziose, la Douve du foie et les protozoaires. A titre d'exemples plus particuliers de parasites, on peut citer les parasites *Toxoplasma gandii* (parasite responsable de la toxoplasmose) et *Trypanosomia cruzi* (parasite responsable de la maladie de Chagas).

Dans le cadre de l'invention, la détection d'anticorps est réalisée *in vitro.* Les anticorps détectés peuvent être des immunoglobulines IgM et/ou IgG et/ou IgE et/ou IgA.

Le procédé selon l'invention concerne la détection d'anticorps chez des sujets immunodéprimés. Par « sujet», on entend un sujet animal ou humain. Par « sujet immunodéprimé », on entend un sujet qui présente un déficit immunitaire, par rapport à un patient dit normal. Un sujet immunodéprimé appartient, en général, à l'une ou l'autre des catégories suivantes :
- sujets présentant un déficit immunitaire congénital. Il peut s'agir :
   - d'un déficit dit pur, correspondant notamment à un déficit primitif en CD4 ou CD8, un déficit en IL-2 ou un déficit en transduction du signal,
   - d'un déficit dit combiné, correspondant notamment à une mutation de la chaîne gamma du récepteur de l'IL-2, à un déficit d'expression des molécules CMH-II, à une ataxie-télangiectasie (syndrome de Louis-Bar), à un déficit en enzymes RAG ou en protéine Artemis nécessaire(s) à la recombinaison VDJ,
   - d'un déficit sélectif en IgA ou IgG2,
   - d'un syndrome hyper IgM,
   - d'une hypo- ou agammaglobulinémie liée au sexe ou maladie de Bruton,
   - d'une hypogammaglobulinémie commune d'expression variable,
   - d'un déficit sélectif en immunoglobulines,
   - d'une microdélétion 22q11 ou du syndrome de Di George,
   - du syndrome de Hong et Good,
   - du syndrome de Nezelof,
   - d'un déficit en purine nucléoside phosphorylase,
   - d'un déficit isolé en Lymphocytes T,
   - d'un déficit immunitaire combiné sévère par déficit en adénosine désaminase,
   - d'un syndrome des lymphocytes dénudés,
   - d'une amégacaryocytose congénitale avec anomalie du développement des lignées T et B,
   - du syndrome de Wiskott-Aldrich,
- sujets présentant un déficit immunitaire commun variable. Il peut s'agir notamment :
   - d'une neutropénie cyclique,
   - du syndrome de Shwachman-Diamond,
   - du syndrome myélo-dysplasique,
   - d'un déficit chimiotactisme, tel que la maladie de Chediak-Higashi ou le syndrome d'hyperlgE (Buckley),
   - d'un déficit de phagocytose,
   - d'un déficit bactéricide, tels qu'une granulomatose septique chronique ou un déficit myélopéroxydase,
   - déficit de l'axe interféron gamma/interleukine 12,
- sujets présentant une immunosénescence : concerne les hommes et femmes de plus de 65 ans (déclin naturel de la réactivité immunitaire), ou connaissant un stress oxydatif.
- sujets présentant un stress traumatique,
- sujets présentant un ou des désordres métaboliques affectant le système immunitaire.

Parmi, de tels désordres, on peut citer :
- la malnutrition protéino-calorique,
- le déficit en oligo-éléments et vitamines,
- l'insuffisance rénale chronique, syndrome néphrotique,
- les diabètes,
- l'inflammation chronique telle que les désordres intestinaux, articulaires, ou la polyarthrite,
- la sarcoïdose, maladie non maligne du système lymphoïde (déficit cellulaire),
- les hémopathies malignes telles que la leucémie aiguë, la leucémie lymphoïde chronique, le lymphome malin, le myélome multiple et la maladie de Waldenström,
- les anomalies thymiques telles que le thymome (tumeur bénigne du thymus) et l'hypoplasie thymique,
- le déficit de l'activité du macrophage et du polynucléaire, tels que le granulomatose septique, le déficit en myéloperoxydase, le syndrome de Chediak Higashi, le dysfonctionnement de l'actine et le syndrome de Shwachman,
- le déficit en protéines du complément,
- les néoplasies (cancers),
- la séropositivité HIV (SIDA),

- sujets ayant subi ou étant sous traitement allopathique inducteur d'un déficit immunitaire, tel qu'une corticothérapie, un traitement inhibiteur du TNF alpha (dit anti-TNF alpha), un traitement inhibiteur de l'un des interférons, une chimiothérapie, une radiothérapie, un traitement immunosuppresseur préparatoire à une allogreffe, un traitement immunomodulateur post-greffe ou un traitement immunosuppresseur des maladies autoimmunes, et
- sujets ayant subi une splénectomie chirurgicale.

Les déficits immunitaires congénitaux sont rares, hétérogènes, précoces et graves. Leur prévalence est de 1 / 50 000. Les seuls traitements sont l'allogreffe et la thérapie génique. De tels sujets sont, par exemple, les enfants diagnostiqués avec un déficit immunitaire congénital ou « l'enfant bulle » ayant besoin d'une greffe de moelle osseuse compatible, susceptible de restaurer une compétence immunologique.

Les déficits immunitaires communs variables sont les plus fréquents des DIP (déficits immunitaires primitifs correspondant à un ensemble de plus de 200 maladies dues au dysfonctionnement de certaines composantes du système immunitaire de l'adulte. Leur prévalence est variable en fonction des ethnies et est de 1/ 20 000 en France).

En 2014, 8,3% de la population mondiale avait 65 ans ou plus. Ce pourcentage qui va augmenter dans les années à venir, va faire augmenter les cas d'immunosénescence.

La détermination de la réponse humorale, et donc la détection d'anticorps, est de haute importance dans le cas de patients immunodéprimés. La détection d'anticorps est classiquement réalisée sur un échantillon biologique issu d'un sujet d'intérêt, après dilution dudit échantillon. L'apport du procédé selon l'invention est de mettre à disposition de l'utilisateur pour une trousse ou un dispositif de détection d'anticorps dirigés contre un agent infectieux, au moins deux rapports de dilution pour obtenir un échantillon dit dilué soumis à la détection : l'un correspondant à une population dite non immunodéprimée, l'autre correspondant à une population dite immunodéprimée. En fonction des informations connues sur le sujet dont est issu l'échantillon biologique, le rapport de dilution adapté au caractère immunodéprimé ou non du sujet, sera sélectionné et utilisé. Lorsque l'échantillon provient d'un sujet immunodéprimé, le rapport de dilution correspondant à une population dite immunodéprimée sera utilisé.

Le procédé de détermination chez un sujet immunodéprimé d'une réponse humorale due à la présence d'un agent infectieux cible selon l'invention comprend donc les caractéristiques suivantes :
- disposer d'une trousse ou d'un dispositif de détection d'anticorps dirigés contre ledit agent infectieux, capable donc de déterminer la réponse humorale due à la présence dudit agent infectieux cible chez un sujet,
- disposer d'un diluant pour diluer les échantillons à tester, ledit diluant pouvant faire partie de la trousse ou du dispositif de détection,
- disposer d'un rapport de dilution **R2** qui est égal à **V2/(V2+D2)** adapté à une population de sujets non immunodéprimés,
- disposer d'un rapport de dilution **R1** qui est égal à **V1/(V1+D1)** adapté à une population de sujets immunodéprimés, avec **R1** > **R2** et 1,5**R2** ≤ **R1** ≤ 5**R2,**
- choisir le rapport de dilution **R1,** compte tenu du caractère immunodéprimé du sujet dont est issu l'échantillon à analyser.

L'invention a également pour objet un procédé de détermination d'une réponse humorale due à la présence d'un agent infectieux cible à partir d'un échantillon biologique d'un sujet immunodéprimé ou non immunodéprimé, dans lequel le rapport de dilution de l'échantillon est choisi en fonction du caractère immunodéprimé ou non du sujet concerné. Plus précisément, l'invention concerne également un procédé de détermination d'une réponse humorale due à la présence d'un agent infectieux cible chez un sujet immunodéprimé ou non immunodéprimé, par détection dans un échantillon biologique dudit sujet, d'au moins un anticorps cible susceptible d'être produit par ledit sujet lorsque ce dernier est infecté ou a été infecté par ledit agent infectieux cible, caractérisé en ce qu'il présente les caractéristiques suivantes :
- disposer d'une trousse ou dispositif de détection d'anticorps dirigés contre un agent infectieux, capable donc de déterminer la réponse humorale due à la présence dudit agent infectieux cible chez un sujet,
- disposer d'un diluant pour diluer les échantillons à tester, ledit diluant pouvant faire partie de la trousse ou du dispositif de détection,
- disposer d'un rapport de dilution **R2** qui est égal à **V2/(V2+D2)** adapté à une population de sujets non immunodéprimés,
- disposer d'un rapport de dilution **R1** qui est égal à **V1/(V1+D1)** adapté à une population de sujets immunodéprimés, avec **R1** > **R2** et 1,5**R2** ≤ **R1** ≤ 5**R2**,
- choisir le rapport de dilution utilisé en fonction du caractère immunodéprimé ou non du sujet dont est issu l'échantillon à analyser : **R1** est utilisé si le sujet est immunodéprimé et **R2** est utilisé si le sujet est non immunodéprimé.

Dans le cadre de l'invention, il est proposé d'adapter le rapport de dilution utilisé dans le cas d'un échantillon issu d'un sujet immunodéprimé, de manière à ce que celui-ci diffère de celui utilisé dans le cas d'un échantillon issu d'un sujet non immunodéprimé. Selon l'invention, un volume **V1** d'un échantillon **E1** à tester issu d'un sujet immunodéprimé est dilué avec un volume **D1** de diluant, selon un rapport de dilution **R1** qui est égal à **V1/(V1+D1),** le rapport de dilution **R1** étant supérieur au rapport de dilution **R2** utilisé dans le cas d'un sujet non immunodéprimé. **R2** est égal à **V2/(V2+D2)** avec **V2** et **D2** qui sont respectivement le volume d'échantillon **E2** et le volume de diluant utilisé dans le cas d'un sujet non immunodéprimé. Par contre, le diluant utilisé est le même que l'on soit dans le cas d'un échantillon **E1** à tester issu d'un sujet immunodéprimé ou d'un échantillon **E2** issu d'un sujet non immunodéprimé. Un échantillon dilué est ainsi obtenu et c'est sur ce dernier que la détection d'au moins un anticorps cible va être mise en oeuvre, de manière à pouvoir conclure à la présence ou non de la réponse humorale recherchée chez ledit sujet. La détection peut être réalisée sur la totalité de l'échantillon dilué obtenu ou sur une partie seulement de celui-ci.

Le sujet duquel est issu l'échantillon est un sujet humain ou animal. Bien entendu, le sujet immunodéprimé dont est issu l'échantillon **E1** et le sujet non immunodéprimé dont est issu l'échantillon **E2** appartiendront à la même espèce : en particulier, on utilisera pour une trousse ou un dispositif destiné à un sujet humain, un échantillon humain, et pour une trousse ou un dispositif destiné à un animal, un échantillon d'animal de la même espèce.

Dans le cadre de l'invention, le rapport de dilution **R1** dans le cas d'un échantillon **E1** à tester issu d'un sujet immunodéprimé est défini comme suit : 1,5**R2** ≤ **R1** ≤ **5R2, R2** étant le rapport de dilution utilisé dans le cas d'un échantillon **E2** issu d'un sujet non immunodéprimé. Les rapports de dilution **R1** et **R2** seront, le plus souvent, choisis dans la gamme allant de 0,01 à 0,9.

De manière classique, le diluant utilisé pour réaliser la dilution de l'échantillon biologique à tester est une solution liquide qui n'altère pas la réactivité immunologique des anticorps recherchés. A titre d'exemples de diluants pouvant être utilisés, on peut citer les sérums d'animaux tels que le sérum de veau, de cheval ou de mouton, les tampons, notamment Tris, phosphate ou HEPES, ou encore l'eau. Quel que soit le diluant utilisé, celui-ci pourra inclure un ou plusieurs additifs, par exemple, choisis parmi : les polymères, tels que les PEG, les détergents tels que les Tween^{®}, les protéines, les sucres et les lipides, et/ou un ou plusieurs réactifs permettant d'assurer la détection, en particulier un partenaire de liaison pour l'anticorps à détecter, et par exemple un partenaire de liaison marqué comme expliqué plus loin.

Selon un premier mode de réalisation, l'augmentation du rapport de dilution **R1** utilisé dans le cas d'un échantillon **E1** à tester issu d'un sujet immunodéprimé, par rapport au rapport de dilution **R2** utilisé dans le cas d'un échantillon **E2** issu d'un sujet non immunodéprimé, est obtenue par augmentation du volume **V1** d'échantillon **E1,** par rapport au volume **V2** d'échantillon **E2** utilisé dans le cas d'un sujet non immunodéprimé.

Selon un second mode de réalisation, l'augmentation du rapport de dilution **R1** utilisé dans le cas d'un échantillon **E1** à tester issu d'un sujet immunodéprimé, par rapport au rapport de dilution **R2** utilisé dans le cas d'un échantillon **E2** issu d'un sujet non immunodéprimé, est obtenue par diminution du volume **D1** de diluant, par rapport au volume **D2** de diluant utilisé dans le cas d'un sujet non immunodéprimé.

L'homme du métier pourra déterminer les rapports volume d'échantillon/volume de diluant, en fonction du dispositif utilisé et de la méthode de détection mise en oeuvre. Par exemple, dans le cas de l'utilisation d'un dispositif VIDAS@, des rapports volume d'échantillon/volume de diluant dans la gamme allant de 1/100 à 50/100 pourront être utilisés.

Le procédé selon l'invention peut être réalisé sur tout type d'échantillon biologique issu d'un sujet (de sang, d'urine, de selles, de salive ...), mais est particulièrement adapté aux échantillons de sang, de sang total, de sérum ou de plasma.

L'échantillon peut être prétraité, préalablement à sa dilution, par exemple pour inactiver l'agent infectieux, pour dissocier les complexes immuns, pour neutraliser des analytes non spécifiques à la réaction, ou pour rendre les anticorps disponibles aux partenaires de liaison utilisés dans le procédé de détermination de la réponse humorale. De tels exemples de prétraitement sont le chauffage, l'acidification, la réduction, l'oxydation, l'utilisation d'un ou plusieurs agents chaotropiques, d'un ou plusieurs agents chélatants, les procédés mécaniques tels que la filtration, la centrifugation ou la sonication et leurs combinaisons.

La détection réalisée à l'étape c) comprend à la fois la ou les réactions d'affinité des anticorps à détecter avec un ou des réactifs de détection et la mise en évidence de ladite réaction. En tant que réactif de détection, on utilisera un partenaire de liaison de l'anticorps à détecter, éventuellement en combinaison avec un autre réactif. La ou les réactions d'affinité pourront être réalisées de manière concomitante à l'étape de dilution, lorsque le diluant comprendra un partenaire de liaison de l'anticorps à détecter. La détection inclut une étape d'incubation entre l'échantillon dilué et un ou des réactifs de détection et peut comprendre également une ou plusieurs étapes de lavage et/ou de révélation pour mettre en évidence la présence d'anticorps.

La détection du ou des anticorps cible peut être réalisée à l'étape c) de manière quantitative : on parle alors de dosage ou quantification, ou de manière qualitative. La détection sera, par exemple, réalisée par un test biochimique, notamment un test immunologique (nommé immunoessai) en utilisant au moins un partenaire de liaison de l'anticorps à détecter.

Par « qualitative », on entend une réponse positive ou négative quant à la présence d'anticorps d'intérêt, éventuellement chiffrée, sans relation avec la concentration exacte en anticorps d'intérêt, puisqu'il n'y a aucune comparaison avec une courbe de calibration comme c'est le cas pour une détection quantitative (voir ci-après).

Par « quantitative », on entend que la concentration d'au moins un anticorps d'intérêt est déterminée. De manière classique, pour déterminer la quantité d'au moins un anticorps dans un échantillon biologique, le signal proportionnel (dans le cas d'un procédé de détection direct) ou inversement proportionnel (dans le cas d'un procédé indirect) à la quantité dudit anticorps, pourra être comparé à une courbe de calibration obtenue au préalable par des techniques largement connues de l'homme du métier. Ainsi, par exemple, la courbe de calibration est obtenue en effectuant un dosage utilisant le même anticorps, ainsi que des quantités croissantes de cet anticorps. Une courbe est ainsi obtenue en mettant en abscisse, la concentration en anticorps d'intérêt, et en ordonnée, le signal correspondant obtenu après dosage.

Dans le cadre de l'invention, il est possible d'utiliser toute trousse ou dispositif adapté à la détection d'anticorps et donc à la détection d'une réponse humorale chez un sujet. En particulier, la détection dudit au moins anticorps cible est réalisée à l'étape c) par un immunoessai, notamment un test ELISA ou ELFA, direct ou indirect.

Bien entendu, le préfixe « immuno » dans le terme « immunoessai », par exemple, n'est pas à considérer dans la présente demande comme indiquant strictement que le partenaire de liaison est nécessairement un partenaire d'origine immunologique. Ainsi, il est connu de parler de test ELISA pour des tests qui utilisent des partenaires de liaison non immunologiques *stricto sensu,* appelés plus largement en anglais « ligand binding assay », que l'on pourrait traduire en langue française par « essai utilisant la liaison à un ligand », alors que le terme « immuno » est inclus dans l'intitulé *in extenso* correspondant à l'acronyme ELISA. Dans un souci de clarté et d'uniformité, le terme « immuno » est employé dans la présente demande pour désigner toute analyse biologique utilisant au moins un partenaire de liaison adapté pour se lier à un anticorps d'intérêt.

Le partenaire de liaison utilisé sera, de préférence, spécifique, c'est-à-dire qu'il sera capable de se lier à l'anticorps d'intérêt, avec une spécificité élevée, de préférence avec une spécificité supérieure à 90%, voire une spécificité de 100%, notamment quand il est capable de se lier de façon exclusive, ou quasi-exclusive, à l'anticorps d'intérêt. Un partenaire de liaison est dit non spécifique lorsque sa spécificité de liaison à l'anticorps d'intérêt est faible et qu'il est alors capable de se lier à d'autres ligands, tels que d'autres anticorps.

Les antigènes sont des exemples de partenaires de liaison des anticorps. On pourra également utiliser comme partenaire de liaison des anticorps anti-immunoglobulines ainsi que leurs fragments ou leurs analogues. A titre d'exemples de fragments d'anticorps, on peut citer les fragments Fab, Fab', F(ab')2 ainsi que les chaînes scFv (de l'anglais « Single chain variable fragment »), dsFv (de l'anglais « Double-stranded variable fragment »). Ces fragments fonctionnels peuvent notamment être obtenus par génie génétique.

Les analogues d'anticorps peuvent être des nanofitines, aptamères ou DAPPins.

Les analogues d'anticorps nanofitines sont de petites protéines qui, comme les anticorps, sont capables de se lier à une cible biologique (et en particulier à un anticorps) permettant ainsi de la détecter, de la capturer ou tout simplement de la cibler au sein d'un organisme.

Les analogues d'anticorps aptamères sont des oligonucléotides, généralement ARN ou ADN, identifiés dans des banques contenant jusqu'à 10¹⁵ séquences différentes, par une méthode combinatoire de sélection in vitro appelée SELEX pour « Systematic Evolution of Ligands by Exponentiel Enrichment » (Ellington AD et Szostak JW., 1990 Nature, 346 : 818-822). La plupart des aptamères sont composés d'ARN, en raison de la capacité de l'ARN à adopter des structures variées et complexes, ce qui permet de créer à sa surface des cavités de géométries variées, permettant de fixer des ligands divers. Il s'agit d'outils biochimiques d'intérêt qui peuvent être utilisés dans des applications biotechnologiques, diagnostiques ou thérapeutiques. Leur sélectivité et leurs propriétés de fixation de ligands sont comparables à celle des anticorps.

Les analogues d'anticorps « DARPins » pour Designed Ankyrin Repeat ProteINS (Boersma YL et Plütckthun A, 2011 Curr. Opin. Biotechnol, 22 : 849-857) sont une autre classe de protéines permettant de mimer les anticorps et de pouvoir se fixer avec une affinité et une sélectivité élevées sur des protéines cibles (et en particulier sur un anticorps). Ils dérivent de la famille des protéines ankyrines qui sont des protéines adaptatrices permettant de fixer les protéines de membrane intégrales au réseau spectrine/actine qui constitue « la colonne vertébrale » de la membrane plasmatique cellulaire. La structure des ankyrines est basée sur la répétition d'un motif d'environ 33 acides aminés et il en est de même des DARPins. Chaque motif a une structure secondaire de type hélice-coude-hélice (« helix-turn-helix »). Les DAPPins contiennent au moins trois, de préférence quatre à cinq motifs répétés et sont obtenus par screening de banques combinatoires.

Les partenaires de liaison spécifiques ou non du ou des anticorps recherché(s), dans le procédé de l'invention, peuvent être utilisés comme réactifs de capture, comme réactifs de détection ou comme réactifs de capture et de détection.

La visualisation des réactions immunologiques, c'est-à-dire de la liaison anticorps/partenaire de liaison, peut être effectuée par tout moyen de détection, en particulier, par marquage ou résonance plasmonique de surface.

Par marquage, on entend la fixation d'un réactif marqueur capable de générer directement ou indirectement un signal détectable. Une liste non limitative de ces réactifs marqueurs consiste en :
- les enzymes qui produisent un signal détectable, par exemple, par colorimétrie, fluorescence, luminescence, comme la peroxydase de raifort, la phosphatase alcaline, la β-galactosidase, la glucose-6-phosphate déshydrogénase,
- les chromophores comme les composés fluorescents, luminescents, colorants,
- les molécules radioactives comme le ³²P, le ³⁵S ou le ¹²⁵I,
- les molécules fluorescentes telles que les Alexa ou les phycocyanines, et
- les électrochimiluminescents tels que des dérivés organométalliques à base d'acridinium ou de ruthénium.

La détection d'au moins un anticorps d'intérêt peut être réalisée de manière directe, c'est-à-dire par marquage du partenaire de liaison, soit de manière indirecte, c'est-à-dire en utilisant un anti-ligand du partenaire de liaison (ligand).

Les couples ligand/anti-ligand sont bien connus de l'homme du métier, ce qui est le cas, par exemple, des couples suivants : biotine/streptavidine, haptène/anticorps, antigène/anticorps, peptide/anticorps, sucre/lectine, polynucléotide/complémentaire du polynucléotide. Dans ce cas, c'est le ligand qui porte le partenaire de liaison de l'anticorps cible. L'anti-ligand peut être détectable directement par les réactifs marqueurs décrits au paragraphe précédent ou être lui-même détectable par un ligand/anti-ligand.

Ces systèmes indirects de détection peuvent conduire, dans certaines conditions, à une amplification du signal. Cette technique d'amplification du signal est bien connue de l'homme du métier, et l'on pourra se reporter aux demandes de brevet antérieures FR 2781802 ou WO 95/08000 de la Demanderesse.

Selon le type de marquage utilisé, l'homme du métier ajoutera des réactifs permettant la visualisation du marquage ou l'émission d'un signal détectable par tout type d'appareil de mesure approprié, comme par exemple, un spectrophotomètre, un spectrofluorimètre, un densitomètre, un luminomètre ou encore une caméra haute définition.

Le partenaire de liaison permettant de détecter l'anticorps que l'on cherche à détecter peut être notamment fixé sur un support solide. De manière connue, le support solide peut se présenter sous toutes formes appropriées telles qu'une plaque, un cône, une bille, la bille étant éventuellement radioactive, fluorescente, magnétique et/ou conductrice, une barrette, un tube de verre, un puits, une feuille, notamment de format papier, une puce, une plaque de micro-titration ou analogues. Lorsque le support est sous la forme de billes, celles-ci ont, le plus souvent, un diamètre allant de la centaine de micromètres au nanomètre. De préférence, le partenaire de liaison fixé sur le support, utilisé comme partenaire de capture, sera spécifique de l'anticorps d'intérêt et le partenaire de liaison utilisé en détection sera spécifique ou non de l'anticorps d'intérêt.

A titre d'exemples de tests immunologiques tels que définis ci-dessus, on peut citer les méthodes « sandwich » telles qu'ELISA, ELFA, IRMA et RIA, les méthodes dites de compétition et les méthodes d'immunodétection directe comme l'immunohistochimie, l'immunocytochimie, le Western-blot et le Dot-blot.

Les méthodes pour la détection d'anticorps utilisant deux partenaires de liaison sont des méthodes sandwich bien connues de l'homme du métier, à savoir :
- une méthode communément appelée sandwich double antigène, utilisant en capture et en détection deux antigènes, de nature identique ou différente, capables d'être reconnus par l'anticorps recherché,
- une méthode communément appelée immunocapture, utilisant en capture un anticorps, un fragment d'anticorps ou un analogue d'anticorps, comme décrit précédemment, et en détection un antigène et
- une méthode communément appelée sandwich indirect, utilisant en capture un antigène et en détection un anticorps, un fragment d'anticorps ou analogue d'anticorps.

L'immunochromatographie, est également nommée immunoessai par flux latéral. Les dispositifs généralement utilisés dans de tels tests comprennent un milieu de diffusion qui permet la migration de l'échantillon liquide, généralement immobilisé sur un support. On distingue classiquement plusieurs zones au niveau du milieu de diffusion qui sont une zone d'application de l'échantillon liquide, une zone de marquage et une zone réactionnelle, cette dernière comprenant, le plus souvent, une zone de visualisation (également nommée zone capture) et une zone de contrôle. Ces différentes zones sont en communication de fluide. Ainsi, l'anticorps cible, s'il est présent dans l'échantillon déposé au niveau de la zone d'application, se lie à un premier partenaire de liaison marqué au niveau de la zone de marquage, le complexe ainsi formé migre ensuite jusqu'à la zone réactionnelle, où il est immobilisé au niveau de la zone de capture par réaction avec un deuxième partenaire de liaison lié au milieu de diffusion, et l'utilisateur peut déterminer si l'anticorps est bien présent par mise en évidence d'un signal détectable, qui est déterminé par le type de marquage associé au premier partenaire de liaison. Généralement, la présence de l'anticorps cible dans l'échantillon est matérialisée sous la forme d'une ligne détectable, habituellement dénommée ligne test. En général, la zone réactionnelle comprend également une zone de contrôle de la migration de l'échantillon qui va indiquer à l'utilisateur que l'échantillon a migré correctement au travers du milieu de diffusion, en amont de la zone de visualisation. Ce peut être, par exemple, par la révélation d'une ligne de contrôle d'une couleur prédéterminée. On peut citer, à titre d'exemples, les demandes de brevet WO 2004/003559, WO 2006/092103, WO 2007/081330, US 2004/0161859 et WO 2012/172232 qui décrivent de tels dispositifs.

En particulier, la détection de l'étape c) sera réalisée grâce à un test de courte durée, que sont notamment les tests ELISA, ELFA et immunochromatographiques. Notamment, la durée entre le début de la dilution de l'étape b) et l'obtention de la conclusion de l'étape c) est inférieure ou égale à 2 heures, de préférence inférieure ou égale à une heure et 30 minutes, et encore préférentiellement inférieure ou égale à une heure. De préférence, cette durée est de 45 min, 30 min, 20 min, 15 min, 10 min ou 5 min. Dans le cas d'un test ELISA/ELFA, on préférera utiliser une durée de 20 à 45 min et dans le cas d'un test immunochromatographique, on préférera utiliser une durée de 5 à 15 min.

De manière avantageuse, le procédé selon l'invention sera réalisé grâce à un test automatisé avec un temps d'incubation défini, contrairement à un test non automatisé du type microplaque, ou sera réalisé par immunochromatographie.

A titre d'exemples de dispositifs (également nommés instruments) pouvant être utilisés pour mettre en œuvre le procédé selon l'invention, on peut citer les dispositifs et instruments de diagnostic *in vitro* suivants : Architect^{®} commercialisé par Abbott diagnostics, Cobas^{®} et Elecsys^{®} commercialisés par Roche Diagnostics, Liaison^{®} commercialisé par Diasorin et VIDAS^{®} commercialisé par bioMérieux qui possèdent tous un menu de tests de diagnostics infectieux. Ces différents instruments comportent un programme dédié à la toxoplasmose / rubéole / CMV / herpès pour le suivi de la femme enceinte, un programme pédiatrie pour le diagnostic d'infections et/ou le suivi de la vaccination pour les virus rougeole / oreillons / varicelle, un programme Hépatite pour le diagnostic d'infections et /ou le suivi de la vaccination pour les virus HBV, HCV, HAV, HEV, un programme HIV pour le diagnostic des infections par les virus HIV. Ils sont également adaptés pour la détection des infections par le virus EBV (mononucléose), la détection des infections par *Borrelia burgdorferi sensu lato* (maladie de Lyme), la détection des infections par *Clostridium difficile,* la détection des infections par *Helicobacter pylori,* la détection des infections par *Mycoplasma pneumoniae,* la détection des infections par le virus du Nil occidental, la détection des infections par *Trypanosomia cruzi* (Chagas), la détection des infections par *Treponema pallidum* (Syphilis), ou encore la détection des infections par le virus de la dengue.

La détection réalisée à l'étape c) conduit à un signal qui peut être détecté. La conclusion apportée à l'étape c) se fait classiquement par comparaison de ce signal à un signal de référence. En particulier, la conclusion quant à l'éventuelle présence de la réponse humorale sera positive si le signal obtenu est supérieur à un signal de référence **S1.**

Par « signal de référence », on entend une valeur ou intervalle de valeurs utilisé pour déterminer si la conclusion du procédé de détermination selon l'invention est positive (il est considéré qu'il y a présence d'une réponse humorale chez le sujet dont est issu l'échantillon) ou négative (il est considéré qu'il y a absence de réponse humorale chez le sujet dont est issu l'échantillon). Le signal de référence pourra être soit une valeur discrète, soit un intervalle de valeurs correspondant à une zone d'indétermination, appelée aussi zone grise. Lorsque le signal obtenu à partir d'un échantillon est supérieur à ce signal de référence (borne supérieure dans le cas d'un intervalle), il est conclu à la présence de la réponse humorale chez le sujet et lorsque le signal obtenu est inférieur à ce signal de référence (borne inférieure dans le cas d'un intervalle), il est conclu à l'absence de la réponse humorale chez le sujet. Bien évidemment, lorsque la valeur mesurée sera incluse dans l'intervalle d'indétermination, ou sera très proche de la valeur de référence dans le cas d'une valeur discrète, on ne pourra pas conclure définitivement et il conviendra de conduire des investigations supplémentaires. De manière préférée, le signal de référence sera une valeur discrète correspondant au seuil de positivité du procédé.

De manière théorique, un sujet n'ayant jamais été en contact avec un agent infectieux donné ne présente pas d'anticorps dirigé contre cet agent infectieux. En réalité, il existe toujours des réactivités croisées non spécifiques dues à la polyvalence de certains anticorps. C'est pourquoi il est nécessaire de déterminer un signal de référence correspondant à un seuil de positivité, spécifique à l'agent infectieux, permettant de donner un statut positif (présence de réponse humorale contre ledit agent infectieux) ou négatif (absence de réponse humorale contre ledit agent infectieux). Ce signal de référence est déterminé sur une population représentative et est choisie, notamment pour correspondre à la valeur qui donnera le meilleur compromis entre sensibilité et spécificité diagnostiques. La sensibilité mesure la capacité d'un test à donner un résultat positif (VP = vrais positifs) dans le cas où des anticorps sont effectivement produits pour lutter contre l'agent infectieux considéré chez un sujet. Une performance de la sensibilité s'accompagne toujours d'une performance de la spécificité. Cette dernière correspond à la capacité d'obtenir un résultat négatif (VN = vrais négatifs) dans le cas d'absence réelle (présente ou passé) de l'agent infectieux considéré chez un sujet.

En traçant les courbes de détection d'anticorps obtenues en fonction du pourcentage de la population « vrai négatif » (spécificité), en parallèle de la population vrai positif (VP), on détermine un seuil de positivité (signal de référence) qui correspond à la jonction des deux courbes. Il existe souvent une zone de superposition des résultats VP et VN, de sorte que dans cet intervalle, les résultats sont rendus équivoques.

La position du seuil de positivité peut être définie en déterminant le meilleur couple sensibilité / spécificité c'est-à-dire celui pour lequel le nombre de FP et FN (faux résultats positifs et négatifs) est le plus faible. Le seuil de positivité d'un test influence sa sensibilité et sa spécificité. Ainsi, avec un seuil abaissé, un test est plus sensible mais moins spécifique.

Les échantillons utilisés pour déterminer le signal de référence sont des échantillons biologiques de même nature que celle de l'échantillon biologique à tester ou tout du moins d'une nature compatible pour constituer une référence quant à la détection des anticorps cibles.

Ensemble, les performances de sensibilité et spécificité d'un test de diagnostic caractérisent sa pertinence intrinsèque. Ces performances sont vérifiées durant le développement d'un test, puis, validées lors d'essais cliniques sur des collections d'échantillons recrutés par les experts dans la population dite normale, c'est-à-dire, de préférence, dans le cas présent, dans une population non immunodéprimée. Les performances sont mentionnées dans la notice d'un test commercial et l'obtention de résultats en concordance des performances du test est liée aux conditions de sa mise en œuvre : volume d'échantillon, rapport de dilution de l'échantillon analysé durant le temps attribué à l'incubation réactionnelle, etc..

La position du signal de référence (seuil de positivité) peut dépendre de l'utilisation attribuée au test : un test très sensible est surtout utile pour le dépistage précoce d'une infection ou infestation (peu de FN), alors qu'un test très spécifique est utile pour s'assurer qu'une infection ou infestation est absente, pour les suivis vaccinaux notamment (peu de FP). L'homme du métier pourra donc adapter le signal de référence sélectionné en fonction de l'application visée par le procédé selon l'invention.

Cependant, l'équilibre défini dans le test n'est adapté qu'aux sujets de système immunitaire dit normal (non immunodéprimés), il est moins pertinent pour les sujets immunodéprimés. Le procédé selon l'invention propose de pallier à ce problème en ajustant le rapport de dilution, dans le cas d'un sujet immunodéprimé.

De plus, selon un mode de réalisation particulier du procédé de détermination d'une réponse humorale due à la présence d'un agent infectieux cible chez un sujet immunodéprimé selon l'invention, il est également proposé que le signal obtenu à l'étape c) soit comparé à un signal de référence spécifique pour une population de sujets immunodéprimés. En particulier, la conclusion quant à l'éventuelle présence de la réponse humorale sera positive si le signal obtenu est supérieur à un signal de référence **S1,** ledit signal de référence **S1** étant supérieur ou égal au signal de référence **S2** utilisé dans le cas d'un échantillon issu d'un sujet non immunodéprimé.

En d'autres termes, le procédé selon l'invention comprendra alors également les caractéristiques suivantes :
- disposer d'un signal de référence **S2** adapté à une population de sujets non immunodéprimés,
- disposer d'un signal de référence **S1** adapté à une population de sujets immunodéprimés, avec **S1** ≥ **S2,**
- choisir le signal de référence **51** utilisé pour apporter une conclusion à l'étape c), du fait du caractère immunodéprimé du sujet dont est issu l'échantillon testé.

Dans le cas du procédé de détermination d'une réponse humorale due à la présence d'un agent infectieux cible chez un sujet immunodéprimé ou non, selon l'invention, ce dernier comprendra, selon un mode de réalisation particulier, les caractéristiques suivantes :
- disposer d'un signal de référence **S2** adapté à une population de sujets non immunodéprimés,
- disposer d'un signal de référence **S1** adapté à une population de sujets immunodéprimés, avec **S1** ≥ **S2,**
- choisir le signal de référence utilisé pour apporter une conclusion à l'étape c) en fonction du caractère immunodéprimé ou non du sujet dont est issu l'échantillon testé : **S1** est utilisé si le sujet est immunodéprimé et **S2** est utilisé si le sujet est non immunodéprimé.

Le signal de référence **S2** sera alors déterminé à partir de résultats obtenus sur une population de sujets non immunodéprimés, alors que le signal de référence **S1** sera déterminé à partir de résultats obtenus sur une population de sujets immunodéprimés.

Dans la cadre de l'invention, il est donc possible d'utiliser une même trousse ou un même dispositif, en particulier adapté à un procédé automatisé, pour effectuer un diagnostic de la réponse humorale sur population normale et sur population immunodéprimée. Dans ce contexte de double utilisation, le rapport de dilution, voire le signal de référence (seuil de positivité), est(sont) adapté(s), en fonction de l'origine de l'échantillon testé : sujet immunodéprimé ou non immunodéprimé (normal).

Selon l'invention, la détection dudit au moins anticorps cible est réalisée en utilisant une trousse ou un dispositif de diagnostic adapté ou initialement destiné à la détection dudit au moins anticorps cible dans un échantillon biologique provenant d'une population de sujets non immunodéprimés. Il n'est pas nécessaire de procéder à une quelconque adaptation de la trousse ou du dispositif pour pouvoir réaliser la détection dudit au moins anticorps cible dans un échantillon provenant d'un sujet immunodéprimé, hormis celle(s) prévue(s) dans le cadre de l'invention, à savoir une augmentation du rapport de dilution, accompagné éventuellement d'une augmentation du signal de référence définissant le seuil de positivité et donc la présence d'une réponse humorale. La détection réalisée à l'étape c) utilise la même trousse ou le même dispositif, et en particulier les mêmes réactifs que ceux utilisés dans le cas d'une détection réalisée à partir d'un échantillon provenant d'un sujet dit normal ou non immunodéprimé.

De manière avantageuse, dans le cadre de l'invention, la détection dudit au moins anticorps est réalisée à l'étape c) dans les mêmes conditions que dans le cas d'une détection réalisée à partir d'un échantillon biologique **E2** d'un sujet non immunodéprimé. Il est néanmoins possible de faire varier certaines conditions, en fonction de l'échantillon (issu d'un sujet immunodéprimé ou non immunodéprimé), notamment la durée entre le début de la dilution de l'étape b) et l'obtention de la conclusion de l'étape c) et notamment la durée d'incubation, ou la température utilisée à l'étape c), et notamment pendant l'incubation.

Dans le cadre de l'invention, la détection et la génération du signal lors de la détection de l'étape c) seront, de préférence, réalisées dans les mêmes conditions que celles utilisées dans le cas d'une détection dudit au moins anticorps cible dans un échantillon provenant d'un sujet non immunodéprimé. De manière avantageuse, seul le rapport de dilution, et éventuellement le signal de référence, est(sont) modifié(s) comme décrit dans le cadre de l'invention, entre une détermination d'une réponse humorale à partir d'un échantillon issu d'un sujet immunodéprimé et une détermination d'une réponse humorale à partir d'un échantillon issu d'un sujet non immunodéprimé.

Les conditions de détection mises en œuvre à l'étape c) seront classiques pour l'homme du métier. En particulier, un volume de l'échantillon dilué de 5 µL à 1 mL, de préférence 10 à 500 µL, et préférentiellement de 20 à 250 µL, pourra être utilisé. La température utilisée pour l'incubation pourra être classiquement dans la gamme allant de 15 à 40°C, de préférence 18 à 39°C et sera, préférentiellement, égale à 37°C.

Le procédé de détermination de la réponse humorale d'un sujet à un agent infectieux, également nommé agent pathogène, proposé dans le cadre de l'invention a notamment les applications suivantes : pour l'aide au diagnostic *in vitro* ou pour le diagnostic *in vitro* d'une infection par l'agent pathogène chez un sujet susceptible d'être infecté, pour le suivi thérapeutique d'un sujet infecté par l'agent pathogène, pour faire des études épidémiologiques de la séroprévalence des anticorps anti-agent pathogène dans une population ou dans un territoire géographique donné, pour déterminer si un sujet a besoin d'être vacciné ou revacciné contre l'agent pathogène (les anticorps recherchés sont pour ce dernier cas, des IgG). De manière stricte, on appelle « infection » une maladie occasionnée par une bactérie, un virus ou un champignon et « infestation » une maladie occasionnée par un parasite. Mais dans le cadre de la présente description, par souci de simplification, le terme infecté englobera le terme infesté puisque les parasites sont qualifiés d'agents infectieux. En particulier, le procédé selon l'invention pourra être utilisé pour l'aide au diagnostic *in vitro* ou pour le diagnostic *in vitro* d'une infection par une bactérie, un virus, un champignon ou un parasite listé dans le cadre de l'invention, ou encore pour le suivi thérapeutique d'un sujet infecté par une bactérie, un virus, un champignon ou un parasite listé dans le cadre de l'invention. Le procédé selon l'invention est particulièrement adapté à la détection d'une réponse humorale, chez un patient immunodéprimé, du fait de la présence (actuelle ou future) d'un agent infectieux choisi parmi les parasites *Taxaplasma gondii* (parasite responsable de la toxoplasmose), *Trypanosomia cruzi* (parasite responsable de la maladie de Chagas), les bactéries *Clostridium difficile, Helicobacter pylori, Mycoplasma pneumoniae, Treponema pallidum* (responsable de la Syphilis), *Borrelia burgdorferi sensu lato* (responsable de la maladie de Lyme), le virus responsable de la rubéole, le cytomegalovirus (CMV), les virus de l'herpès, le virus responsable de la rougeole, le virus responsable des oreillons, le virus responsable de la varicelle, les virus hépatiques : HBV, HCV, HAV et HEV, le virus HIV, le virus EBV (de la mononucléose), le virus du Nil occidental et le virus de la dengue.

L'exemple ci-après permet d'illustrer l'invention mais n'a nullement un caractère limitatif.

### EXEMPLE

### Détection des IgG dirigées contre le virus zona-varicelle (VZV) chez les non immunodéprimés et les immunodéprimés.

La varicelle est une maladie infectieuse très fréquente : 90 % des enfants contractant l'infection avant l'âge de 12 ans. Elle est causée par un virus à ADN de la famille des Herpès viridae, le VZV (Virus Zona - Varicelle), qui se transmet uniquement entre humains. La varicelle représente la primo-infection par le virus VZV et cette infection est immunisante. Le zona est l'expression clinique de la réactivation du VZV. L'immunité acquise suite à la primo-infection ou suite à une vaccination persiste pendant de nombreuses années mais peut baisser en cas d'immunodépression ou encore de manière liée à l'âge.

Dans des populations à risque, il convient de rechercher les IgG anti-VZV afin de pouvoir évaluer leur statut immunitaire. De telles populations à risque sont : les personnels de soin, les femmes enceintes, les nouveau-nés, les immunodéprimés.

### a) Préparation d'une protéine recombinante VGLE du virus zona-varicelle

Le gène ORF68 codant pour la glycoprotéine gE du virus zona-varicelle (N° accession UniProtKB P09259), appelée aussi VGLE a été utilisé pour la construction. Le domaine extra-membranaire (acides aminés 31-538 correspondant à la **SEQ ID N°1)** de la protéine native (623 acides aminés au total) a été fusionné à 6 histidines du côté N-Terminal pour permettre une purification par chromatographie d'affinité métal-chélate. La séquence ainsi choisie a été obtenue sous forme de gène synthétique, cloné dans un vecteur d'expression, puis le plasmide introduit dans des bactéries E. coli. Après culture, les bactériens ont été récupérés, lysés et la protéine recombinante d'intérêt purifiée sur résine Ni-NTA (Qiagen). Après un cycle de lavage, la protéine est éluée en présence d'un gradient imidazole puis dialysée et quantifiée.

SEQ ID N°1 :

SVLRYDDFHT DEDKLDTNSV YEPYYHSDHA ESSWVNRGES SRKAYDHNSP
YIWPRNDYDG FLENAHEHHG VYNQGRGIDS GERLMQPTQM SAQEDLGDDT
GIHVIPTLNG DDRHKIVNVD QRQYGDVFKG DLNPKPQGQR LIEVSVEENH
PFTLRAPIQR IYGVRYTETW SFLPSLTCTG DAAPAIQHIC LKHTTCFQDV
VVDVDCAENT KEDQLAEISY RFQGKKEADQ PWIVVNTSTL FDELELDPPE
IEPGVLKVLR TEKQYLGVYI WNMRGSDGTS TYATFLVTWK GDEKTRNPTP
AVTPQPRGAE FHMWNYHSHV FSVGDTFSLA MHLQYKIHEA PFDLLLEWLY
VPIDPTCQPM RLYSTCLYHP NAPQCLSHMN SGCTFTSPHL AQRVASTVYQ
NCEHADNYTA YCLGISHMEP SFGLILHDGG TTLKFVDTPE SLSGLYVFVV
YFNGHVEAVA YTVVSTVDHF VNAIEERGFP PTAGQPPATT KPKEITPVNP
GTSPLLRY

### b) Mode opératoire de l'immunoessai VIDAS pour détecter les IgG anti-VZV

Le cône à usage unique sert à la fois de phase solide pour la réaction et de système de pipetage. La cartouche est composée de 10 puits (X0 à X9) recouverts d'une feuille d'aluminium scellée et étiquetée. Le premier puits (X0) comporte une partie prédécoupée pour faciliter l'introduction de l'échantillon. Le dernier puits (X9) est une cuvette optique dans laquelle la fluorescence du substrat est mesurée. Les différents réactifs nécessaires à l'analyse sont contenus dans les puits intermédiaires (X1 à X8). Toutes les étapes du test sont réalisées automatiquement par l'instrument. Elles sont constituées d'une succession de cycles d'aspiration/refoulement du milieu réactionnel.

Les cônes ont été sensibilisés avec 300µL d'une solution de la protéine recombinante VGLE décrite en a) et diluée à 3 µg/mL dans un tampon carbonate 77 mM, pH 9,2. Après environ 20h d'incubation à +18/25°C avec la solution de sensibilisation, les cônes sont vidés. Ensuite, 300 µL d'une solution tampon Tris 200 mM contenant 5 g/L de d'albumine sérique bovine (BSA) sont ajoutés. La passivation de la phase se poursuit à +18/25°C sur la nuit. Les cônes sont vidés, séchés, puis conservés à +4°C jusqu'à utilisation, à l'abri de l'humidité.

L'automate VIDAS@ mélange 11,4 µL de l'échantillon de sérum ou de plasma à tester dans 400 µL de diluant échantillon contenant un tampon phosphate pH 7,4 contenant 154 mM de NaCl et 5 g/L de BSA. Dès que le cône VIDAS@ est en contact avec l'échantillon, la première étape de la réaction immunologique commence. Cette étape permet la liaison spécifique des IgG anti-VGLE présents ou non dans l'échantillon de sérum ou de plasma, à la protéine virale VGLE adsorbée sur le cône. Après 210 secondes d'incubation à 37°C, les composants non liés sont éliminés par lavage avec un tampon Tris 200 mM pH 7,8, NaC! 300 mM et Tween 20 0,05%. Lors de la seconde étape, le cône est incubé avec une solution de conjugué contenant environ 20 ng/mL d'une IgG de souris anti-IgG humain (bioMérieux), couplé à la phosphatase alcaline, dans un tampon phosphate 10 mM, NaCl 154 mM, lait en poudre 5 g/L. Le puits X5 contient 400 µL de cette solution que le cône aspire/refoule pendant 4 minutes, toujours à 37°C. La seconde étape entraîne la formation d'un complexe entre les IgG anti-VGLE retenues sur la phase solide et le conjugué anti-IgG couplé à la phosphatase alcaline. Cette étape est suivie de 2 lavages successifs, afin d'éliminer les composés non fixés.

Lors de l'étape finale de révélation, le substrat 4-méthylombelliferyl phosphate est aspiré, puis refoulé dans le cône ; l'enzyme du conjugué catalyse la réaction d'hydrolyse de ce substrat en 4-méthylombelliferone dont la fluorescence émise est mesurée à 450 nm. La valeur du signal de fluorescence (RFV=relative fluorescence value) est proportionnelle à la concentration des IgG anti VZV présents dans l'échantillon.

### et Résultats

Dans les conditions d'utilisation de référence, le volume d'échantillon biologique utilisé est de 11,4 µL. Dans ces conditions, le signal de référence permettant de discriminer les échantillons positifs (présence d'une réponse humorale) des échantillons négatifs (absence d'une réponse humorale) est de 70 RFV.

Afin de gagner en sensibilité diagnostique et de mieux détecter les IgG anti-VZV chez les immunodéprimés, nous avons augmenté le volume d'échantillon de 11,4 µL à 38,3 µL, sans modifier le volume de diluant maintenu à 400 µL ce qui correspond à une augmentation d'un facteur 3,1 du rapport de dilution (volume échantillon)/(volume échantillon + volume diluant). Pour des échantillons faiblement positifs collectés chez des sujets non immunodéprimés, cette augmentation du volume d'échantillon permet d'augmenter le signal obtenu de 215 RFV en moyenne (+93 à + 324 RFV) comme illustré dans le **Tableau 1.** De plus, l'augmentation du signal non spécifique n'est que de +21 RFV en moyenne (+4 à + 57 RFV). Comme le signal spécifique augmente bien plus que le signal non spécifique, la modification du signal de référence a été envisagée.

**Tableau 1. Comparaison des signaux obtenus en augmentant le volume d'échantillon pour des sujets non immunodéprimés.**

| | **Signal VIDAS (RFV)** | | **Signal VIDAS (RFV) "bruit de fond"** | |
|---|---|---|---|---|
| **Code échantillon** | **volume échantillon 11,4 µL** | **volume échantillon 38,3 µL** | **volume échantillon 11,4 µL** | **volume échantillon 38,3 µL** |
| se48-H3 | 147 | 385 | 20 | 77 |
| se48-H8 | 72 | 206 | 9 | 23 |
| se48-H28 | 152 | 476 | 15 | 56 |
| se48-H32 | 97 | 280 | 4 | 8 |
| se48-F5 | 132 | 371 | 20 | 49 |
| se48-FII | 154 | 425 | 28 | 66 |
| se48-F16 | 81 | 263 | 6 | 16 |
| se48-F18 | 177 | 497 | 9 | 17 |
| se48-F31 | 133 | 367 | 18 | 29 |
| se48-F32 | 155 | 425 | 19 | 33 |
| se48-F36 | 117 | 253 | 22 | 59 |
| se48-F40 | 100 | 281 | 13 | 18 |

Ainsi, 47 échantillons obtenus chez des sujets immunodéprimés (patients transplantés, immunodéprimés suite au traitement immunosuppresseur administré pour éviter le rejet du greffon) ont été dosés, afin de déterminer un nouveau signal de référence adaptée à volume d'échantillon de 38,3 µL et une population d'immunodéprimés : le nouveau signal de référence a été fixé à 150 RFV. Ensuite, les deux couples volume d'échantillon/ signal de référence ont été comparés sur un nouveau panel d'échantillons de sujets immunodéprimés. Encore une fois, il s'agit d'un panel d'échantillons faiblement positifs ; la démonstration de la positivité de ces échantillons a été réalisée avec la technique microplaque Enzygnost anti-VZV/IgG à incubation longue. Les résultats sont récapitulés dans le **Tableau 2.**

Parmi ces 32 échantillons faiblement positifs, 6 ne sont pas détectés par le test VIDAS dans les conditions de référence (volume d'échantillon 11,4 µL). Il s'agit donc de faux-négatifs. Dans les conditions de dosage améliorées (volume d'échantillon 38,3 µL), le nombre de faux-négatifs n'est plus que de 4. Ce résultat correspond bien à une amélioration de la sensibilité diagnostique de 81% à 87%. Il est, cependant, important de noter que cette estimation de sensibilité sous-estime la sensibilité réelle du test. En effet, le panel utilisé n'est pas un panel de diagnostic, il est enrichi en échantillons difficiles à détecter.

En conclusion, l'augmentation du volume de prise d'essai et le choix d'un second signal de référence ont permis d'améliorer la sensibilité diagnostique d'un coffret de diagnostic sérologique pour les populations immunodéprimées, sans changer ni les réactifs du coffret, ni leurs concentrations.

**Tableau 2. Comparaison des immunoessais avec volume d'échantillon standard et volume d'échantillon augmenté chez des patients immunodéprimés.**

| | **VIDAS** volume d'échantillon 11,4 µL | | **VIDAS** volume d'échantillon **38,3 µL** | |
|---|---|---|---|---|
| **Code échantillon** | **Signal RFV** | **Interprétation Pos: RFV>70** | **Signal RFV** | **Interprétation Pos : RFV>150** |
| 121850 | 185 | Pos | 489 | Pos |
| 121665 | 245 | Pos | 820 | Pos |
| 121852 | 218 | Pos | 524 | Pos |
| 121860 | 173 | Pos | 491 | Pos |
| 121981 | 78 | Pos | 201 | Pos |
| 121979 | 137 | Pos | 373 | Pos |
| 121986 | 181 | Pos | 430 | Pos |
| 121990 | 181 | Pos | 515 | Pos |
| 121991 | 61 | **Neg** | 127 | **Neg** |
| 121993 | 231 | Pos | 714 | Pos |
| 121994 | 132 | Pos | 297 | Pos |
| 122001 | 283 | Pos | 871 | Pos |
| 122011 | 80 | Pos | 205 | Pos |
| 122015 | 185 | Pos | 444 | Pos |
| 121606 | 132 | Pos | 378 | Pos |
| 127333 | 62 | **Neg** | 211 | Pos |
| 121642 | 65 | **Neg** | 105 | **Neg** |
| 122021 | 34 | **Neg** | 51 | **Neg** |
| 122022 | 59 | **Neg** | 170 | Pos |
| 122025 | 151 | Pos | 456 | Pos |
| 122033 | 127 | Pos | 294 | Pos |
| 122038 | 28 | **Neg** | 109 | **Neg** |
| 127107 | 147 | Pos | 375 | Pos |
| 127091 | 123 | Pos | 308 | Pos |
| 121666 | 255 | Pos | 854 | Pos |
| 43240 | 169 | Pos | 330 | Pos |
| 43255 | 252 | Pos | 649 | Pos |
| 43258 | 266 | Pos | 579 | Pos |
| 43266 | 278 | Pos | 638 | Pos |
| 43290 | 233 | Pos | 498 | Pos |
| 43314 | 261 | Pos | 608 | Pos |
| 43318 | 255 | Pos | 510 | Pos |

## Revendications

1. Procédé de détermination d'une réponse humorale due à la présence d'un agent infectieux cible chez un sujet immunodéprimé, par détection dans un échantillon biologique **E1** dudit sujet, d'au moins un anticorps cible susceptible d'être produit par ledit sujet lorsque ce dernier est infecté ou a été infecté par ledit agent infectieux cible, comprenant les étapes suivantes :
a) disposer d'un volume **V1** de l'échantillon **E1,**
b) diluer le volume **V1,** avec un volume **D1** de diluant, selon le rapport de dilution **R1** qui est égal à **V1/(V1+D1),** le rapport de dilution **R1** étant supérieur au rapport de dilution **R2,** avec 1,5**R2** ≤ **R1** ≤ **5R2, R2** étant le rapport de dilution utilisé dans le cas d'un sujet non immunodéprimé et **R2** étant égal à **V2/(V2+D2)** avec **V2** et **D2** qui sont respectivement le volume d'échantillon **E2** et le volume de diluant utilisés dans le cas d'un sujet non immunodéprimé, de manière à obtenir un échantillon dilué,
c) réaliser la détection dudit au moins anticorps cible sur l'échantillon dilué obtenu, par un test immunologique conduisant à l'obtention d'un signal, et conclure quant à la présence éventuelle de la réponse humorale par comparaison du signal détecté à un signal de référence.

2. Procédé de détermination selon la revendication 1, **caractérisé en ce qu'**il présente les caractéristiques suivantes :
- disposer d'une trousse ou d'un dispositif de détection d'anticorps dirigés contre ledit agent infectieux, capable donc de déterminer la réponse humorale due à la présence dudit agent infectieux cible chez un sujet,
- disposer d'un diluant pour diluer les échantillons à tester, ledit diluant pouvant faire partie de la trousse ou du dispositif de détection,
- disposer d'un rapport de dilution **R2** qui est égal à **V2/(V2+D2)** adapté à une population de sujets non immunodéprimés,
- disposer d'un rapport de dilution **R1** qui est égal à **V1/(V1+D1)** adapté à une population de sujets immunodéprimés, avec **R1** > **R2** et 1,5**R2 ≤ R1 ≤** 5**R2,**
- choisir le rapport de dilution **R1,** compte tenu du caractère immunodéprimé du sujet dont est issu l'échantillon à analyser,
- réaliser la détection dudit au moins anticorps cible sur l'échantillon dilué obtenu, par mise en oeuvre de ladite trousse ou dudit dispositif.

3. Procédé de détermination d'une réponse humorale due à la présence d'un agent infectieux cible chez un sujet immunodéprimé ou non immunodéprimé, par détection dans un échantillon biologique dudit sujet, d'au moins un anticorps cible susceptible d'être produit par ledit sujet lorsque ce dernier est infecté ou a été infecté par ledit agent infectieux cible, **caractérisé en ce qu'**il présente les caractéristiques suivantes :
- disposer d'une trousse ou d'un dispositif de détection d'anticorps dirigés contre un agent infectieux, par un test immunologique, capable donc de déterminer la réponse humorale due à la présence dudit agent infectieux cible chez un sujet,
- disposer d'un diluant pour diluer les échantillons à tester, ledit diluant pouvant faire partie de la trousse ou du dispositif de détection,
- disposer d'un rapport de dilution **R2** qui est égal à **V2/(V2+D2)** adapté à une population de sujets non immunodéprimés, avec **V2** et **D2** qui sont respectivement le volume d'échantillon et le volume de diluant,
- disposer d'un rapport de dilution **R1** qui est égal à **V1/(V1+D1)** adapté à une population de sujets immunodéprimés, avec **R1 > R2** et +1,5**R2 ≤ R1 ≤** 5**R2,** et **V1** et **D1** qui sont respectivement le volume d'échantillon et le volume de diluant,
- choisir le rapport de dilution utilisé en fonction du caractère immunodéprimé ou non du sujet dont est issu l'échantillon à analyser : **R1** est utilisé si le sujet est immunodéprimé et **R2** est utilisé si le sujet est non immunodéprimé, et réaliser avec ledit rapport la dilution de l'échantillon, de manière à obtenir un échantillon dilué,
- réaliser la détection dudit au moins anticorps cible sur l'échantillon dilué obtenu, par mise en oeuvre de ladite trousse ou dudit dispositif conduisant à l'obtention d'un signal, et conclure quant à la présence éventuelle de la réponse humorale par comparaison du signal détecté à un signal de référence.

4. Procédé de détermination selon l'une des revendications 1 à 3, **caractérisé en ce que** l'augmentation du rapport de dilution **R1** par rapport au rapport de dilution **R2** est obtenue par augmentation du volume **V1** d'échantillon **E1,** par rapport au volume **V2** d'échantillon **E2** utilisé dans le cas d'un sujet non immunodéprimé.

5. Procédé de détermination selon l'une des revendications 1 à 3, **caractérisé en ce que** l'augmentation du rapport de dilution **R1** par rapport au rapport de dilution **R2** est obtenue par diminution du volume **D1** de diluant, par rapport au volume **D2** de diluant utilisé dans le cas d'un sujet non immunodéprimé.

6. Procédé de détermination selon la revendication 1 ou 2 ou selon l'une des revendications 4 à 5 rattachées à la revendication 1 ou 2, **caractérisé en ce qu'**à l'étape c), un signal est obtenu et la conclusion quant à l'éventuelle présence de la réponse humorale est positive si le signal obtenu est supérieur à un signal de référence **S1,** ledit signal de référence **S1** étant supérieur ou égal au signal de référence **S2** utilisé dans le cas d'un échantillon issu d'un sujet non immunodéprimé.

7. Procédé de détermination selon l'une des revendications 1, 2 ou 6, ou selon l'une des revendications 4 à 5 rattachées à la revendication 1 ou 2, **caractérisé en ce que** la détection dudit au moins anticorps est réalisée à l'étape c) dans les mêmes conditions que dans le cas d'une détection réalisée à partir d'un échantillon biologique **E2** d'un sujet non immunodéprimé.

8. Procédé de détermination selon l'une des revendications 1 à 7, **caractérisé en ce que** la détection dudit au moins anticorps cible est réalisée par un test ELISA ou ELFA, direct ou indirect.

9. Procédé de détermination la revendication 1, 2, 6 ou 7, ou selon l'une des revendications 4 à 5 et 8 rattachées à la revendication 1 ou 2, **caractérisé en ce que** la durée entre le début de la dilution de l'étape b) et l'obtention de la conclusion de l'étape c) est inférieure ou égale à 2 heures, de préférence inférieure ou égale à une heure et 30 minutes, et encore, préférentiellement, inférieure ou égale à une heure.

10. Procédé de détermination selon l'une des revendications 1 à 9, **caractérisé en ce que** l'agent infectieux est une bactérie, un virus, un champignon ou un parasite.

## Patentansprüche

1. Verfahren zur Bestimmung einer humoralen Antwort aufgrund des Vorhandenseins eines Ziel-Infektionserregers bei einer immungeschwächten Person durch Nachweis wenigstens eines Zielantikörpers in einer biologischen Probe E1 der Person, welcher in der Lage ist, von der Person produziert zu werden, wenn letztere mit dem Ziel-Infektionserreger infiziert ist oder infiziert wurde, umfassend die folgenden Schritte:
a) Verfügen über ein Volumen V1 der Probe E1,
b) Verdünnen des Volumens V1 mit einem Volumen D1 eines Verdünnungsmittels entsprechend dem Verdünnungsverhältnis R1, das gleich V1/(V1+D1) ist, wobei das Verdünnungsverhältnis R1 größer als das Verdünnungsverhältnis R2 ist, wobei 1,5R2 **≤** R1 ≤ 5R2, wobei R2 das Verdünnungsverhältnis ist, welches im Fall einer nicht immungeschwächten Person verwendet wird und R2 gleich V2/(V2+D2) ist, wobei V2 und D2 das Probenvolumen E2 bzw. das Verdünnungsmittelvolumen sind, welche im Fall einer nicht immungeschwächten Person verwendet werden, um eine verdünnte Probe zu erhalten,
c) Durchführen des Nachweises des wenigstens Zielantikörpers an der erhaltenen verdünnten Probe durch einen immunologischen Test, der zum Erhalten eines Signals führt, und Schließen auf das mögliche Vorliegen der humoralen Antwort durch Vergleichen des nachgewiesenen Signals mit einem Referenzsignal.

2. Bestimmungsverfahren nach Anspruch 1, **dadurch gekennzeichnet, dass** es die folgenden Merkmale aufweist:
- Verfügen über ein Kit oder eine Vorrichtung zum Nachweis von Antikörpern, die gegen den Infektionserreger gerichtet sind, welche(s) folglich in der Lage ist, die humorale Antwort aufgrund des Vorhandenseins des Ziel-Infektionserregers bei einer Person zu bestimmen,
- Verfügen über ein Verdünnungsmittel zum Verdünnen der zu testenden Proben, wobei das Verdünnungsmittel Teil des Nachweiskits oder der Nachweisvorrichtung sein kann,
- Verfügen über ein Verdünnungsverhältnis R2, das gleich V2/(V2+D2) ist und für eine Population von nicht immungeschwächten Personen geeignet ist,
- Verfügen über ein Verdünnungsverhältnis R1, das gleich V1/(V1+D1) ist und für eine Population von immungeschwächten Personen geeignet ist, wobei R1 > R2 und 1,5R2 ≤ R1 ≤ 5R2,
- Auswählen des Verdünnungsverhältnisses R1 unter Berücksichtigung der immungeschwächten Natur der Person, von der die zu analysierende Probe stammt,
- Durchführen des Nachweises des wenigstens Zielantikörpers an der erhaltenen verdünnten Probe durch Verwendung des Kits oder der Vorrichtung.

3. Verfahren zur Bestimmung einer humoralen Antwort aufgrund des Vorhandenseins eines Ziel-Infektionserregers bei einer immungeschwächten oder nicht immungeschwächten Person durch Nachweis wenigstens eines Zielantikörpers in einer biologischen Probe der Person, welcher in der Lage ist, von der Person produziert zu werden, wenn letztere mit dem Ziel-Infektionserreger infiziert ist oder infiziert wurde, **dadurch gekennzeichnet, dass** es die folgenden Eigenschaften aufweist:
- Verfügen über ein Kit oder eine Vorrichtung zum Nachweis von Antikörpern, die gegen einen Infektionserreger gerichtet sind, durch einen immunologischen Test, welche(s) folglich in der Lage ist, die humorale Antwort aufgrund des Vorhandenseins des Ziel-Infektionserregers bei einer Person zu bestimmen,
- Verfügen über ein Verdünnungsmittel zum Verdünnen der zu testenden Proben, wobei das Verdünnungsmittel Teil des Nachweiskits oder der Nachweisvorrichtung sein kann,
- Verfügen über ein Verdünnungsverhältnis R2, das gleich V2/(V2+D2) ist und für eine Population von nicht immungeschwächten Personen geeignet ist, wobei V2 und D2 das Probenvolumen bzw. das Verdünnungsmittelvolumen sind,
- Verfügen über ein Verdünnungsverhältnis R1, das gleich V1/(V1+D1) ist und für eine Population von immungeschwächten Personen geeignet ist, wobei R1 > R2 und 1,5R2 < R1 < 5R2, und V1 und D1 das Probenvolumen bzw. das Verdünnungsmittelvolumen sind,
- Auswählen des Verdünnungsverhältnisses, das in Abhängigkeit davon verwendet wird, ob die Person, von der die zu analysierende Probe stammt, immungeschwächt ist oder nicht: R1 wird verwendet, wenn die Person immungeschwächt ist, und R2 wird verwendet, wenn die Person nicht immungeschwächt ist, und Durchführen der Verdünnung der Probe mit dem genannten Verhältnis, um eine verdünnte Probe zu erhalten,
- Durchführen des Nachweises des wenigstens Zielantikörpers an der erhaltenen verdünnten Probe durch Verwenden des Kits oder der Vorrichtung, was zum Erhalten eines Signals führt, und Schließen auf das mögliche Vorliegen der humoralen Antwort durch Vergleichen des nachgewiesenen Signals mit einem Referenzsignal.

4. Bestimmungsverfahren nach einem der Ansprüche 1 bis 3, **dadurch gekennzeichnet, dass** die Erhöhung des Verdünnungsverhältnisses R1 gegenüber dem Verdünnungsverhältnis R2 durch Erhöhen des Volumens V1 der Probe E1 gegenüber dem Volumen V2 der Probe E2 erreicht wird, das im Fall einer nicht immungeschwächten Person verwendet wird.

5. Bestimmungsverfahren nach einem der Ansprüche 1 bis 3, **dadurch gekennzeichnet, dass** die Erhöhung des Verdünnungsverhältnisses R1 gegenüber dem Verdünnungsverhältnis R2 durch Reduzieren des Volumens V1 des Verdünnungsmittels gegenüber dem Volumen D2 des Verdünnungsmittels erreicht wird, das im Fall einer nicht immungeschwächten Person verwendet wird.

6. Bestimmungsverfahren nach Anspruch 1 oder 2 oder nach einem der auf Anspruch 1 oder 2 rückbezogenen Ansprüche 4 bis 5, **dadurch gekennzeichnet, dass** in Schritt c) ein Signal erhalten wird und das Schließen auf das mögliche Vorliegen der humoralen Antwort positiv ist, wenn das erhaltene Signal größer als ein Referenzsignal S1 ist, wobei das Referenzsignal S1 größer als das oder gleich dem Referenzsignal S2 ist, das im Fall einer von einer nicht immungeschwächten Person stammenden Probe verwendet wird.

7. Bestimmungsverfahren nach einem der Ansprüche 1, 2 oder 6, oder nach einem der auf Anspruch 1 oder 2 rückbezogenen Ansprüche 4 bis 5, **dadurch gekennzeichnet, dass** der Nachweis des wenigstens Antikörpers in Schritt c) unter den gleichen Bedingungen wie im Falle eines Nachweises durchgeführt wird, der anhand einer biologischen Probe E2 von einer nicht immungeschwächten Person durchgeführt wird.

8. Bestimmungsverfahren nach einem der Ansprüche 1 bis 7, **dadurch gekennzeichnet, dass** der Nachweis des wenigstens Zielantikörpers durch einen ELISA- oder ELFA-Test direkt oder indirekt durchgeführt wird.

9. Bestimmungsverfahren nach Anspruch 1, 2, 6 oder 7, oder nach einem der auf Anspruch 1 oder 2 rückbebezogenen Ansprüche 4 bis 5 und 8, **dadurch gekennzeichnet, dass** die Zeitdauer zwischen dem Beginn der Verdünnung des Schrittes b) und dem Erhalten der Schlussfolgerung des Schrittes c) weniger als oder gleich 2 Stunden beträgt, vorzugsweise weniger als oder gleich eine Stunde und 30 Minuten beträgt und weiterhin vorzugsweise weniger als oder gleich eine Stunde beträgt.

10. Bestimmungsverfahren nach einem der Ansprüche 1 bis 9, **dadurch gekennzeichnet, dass** der Infektionserreger ein Bakterium, ein Virus, ein Pilz oder ein Parasit ist.

## Claims

1. A determination method for determining, in an immunodepressed subject, a humoral response due to the presence of a target infectious agent, by detection, in a biological sample E1 of said subject, of at least one target antibody that is susceptible of being produced by said subject when the latter is infected with or has been infected with said target infectious agent, the method comprising the following steps:
a) providing a volume V1 of the sample E1;
b) diluting the volume V1 with a volume D1 of diluent, with a dilution ratio R1 that is equal to V1/(V1+D1), the dilution ratio R1 being higher than a dilution ratio R2, with 1.5R2 ≤ R1 ≤ 5R2, R2 being the dilution ratio used for a non-immunodepressed subject, and R2 being equal to V2/(V2+D2), with V2 and D2 respectively being the volume of the sample E2 and the volume of diluent used for a non-immunosuppressed subject, in a manner such as to obtain a diluted sample;
c) detecting said at least one target antibody on the resulting diluted sample, by an immulogical assay leading to the obtaining of a signal and drawing a conclusion concerning the possible presence of the humoral response by comparion of the detected signal to a reference signal.

2. A determination method according to claim 1, **characterized in that** it has the following characteristics:
- providing a kit or device for detecting antibodies directed against said infectious agent, which is thus capable of determining the humoral response due to the presence of said target infectious agent in a subject;
- providing a diluent in order to dilute the test samples, said diluent possibly forming part of the kit or detection device;
- providing a dilution ratio R2 that is equal to V2/(V2+D2), adapted to a population of non-immunodepressed subjects;
- providing a dilution ratio R1 that is equal to V1/(V1+D1), adapted to a population of immunodepressed subjects, with R1 > R2 and 1.5R2 ≤ R1 ≤ 5R2;
- selecting the dilution ratio R1, taking into account the immunodepressed condition of the subject from whom the sample to be analyzed has been obtained,
- carrying out the detection of said at least one target antibody on the obtained diluted sample, by using the said kit or device.

3. A determination method for determining a humoral response due to the presence of a target infectious agent in an immunodepressed or non-immunodepressed subject, by detecting, in a biological sample of said subject, at least one target antibody that is susceptible of being produced by said subject when the latter is infected or has been infected by said target infectious agent, the method being **characterized in that** it has the following characteristics:
- providing a kit or device for detecting antibodies directed against an infectious agent, by an immunological assay, which is thus capable of determining the humoral response due to the presence of said target infectious agent in a subject;
- providing a diluent in order to dilute the test samples, said diluent possibly forming part of the kit or detection device;
- providing a dilution ratio R2 that is equal to V2/(V2+D2), adapted to a population of non-immunodepressed subjects, with V2 and D2 which are respectively the volume of the sample and the volume of diluant;
- providing a dilution ratio R1 that is equal to V1/(V1+D1), adapted to a population of immunodepressed subjects, with R1 > R2 and 1.5R2 ≤ R1 ≤ 5R2 and V1 and D1 which are respectively the volume of the sample and the volume of diluant selecting the dilution ratio used as a function of the immunodepressed condition or otherwise of the subject from whom the sample to be analyzed has been obtained: R1 is used if the subject is immunodepressed and R2 is used if the subject is not immunodepressed and carrying out the dilution of the sample with said dilution ratio, so as to obtain a diluted sample,
- carrying out the detection of said at least one target antibody on the obtained diluted sample, by using the said kit or device leading to the obtaining of a signal, and drawing a conclusion concerning the possible presence of the humoral response by comparion of the detected signal to a reference signal.

4. A determination method according to any one of claims 1 to 3, **characterized in that** the increase in the dilution ratio R1 with respect to the dilution ratio R2 is obtained by increasing the volume V1 of the sample E1 compared with the volume V2 of the sample E2 used for a non-immunodepressed subject.

5. A determination method according to any one of claims 1 to 3, **characterized in that** the increase in the dilution ratio R1 with respect to the dilution ratio R2 is obtained by increasing the volume D1 of diluent compared with the volume D2 of diluent used for a non-immunodepressed subject.

6. A determination method according to claim 1 or 2 or according to any one of claims 4 to 5 attached to claim 1 or 2, **characterized in that** in step c), a signal is obtained and the conclusion as to the possible presence of the humoral response is positive if the signal obtained is higher than a reference signal S1, said reference signal S1 being greater than or equal to a reference signal S2 used for a sample obtained from a non-immunodepressed subject.

7. A determination method according to claim 1, 2 or 6, or according to any one of claims 4 to 5 attached to claim 1 or 2, **characterized in that** the detection of said at least one antibody is carried out in step c) under the same conditions as for detection carried out starting from a biological sample E2 from a non-immunodepressed subject.

8. A determination method according to any one of claims 1 to 7, **characterized in that** the detection of said at least one target antibody is carried out by a direct or indirect ELISA or ELFA test.

9. A determination method claim 1, 2, 6 or 7 or according to any one of claims 4 to 5 and 8 attached to claim 1 or 2, **characterized in that** the period between the start of the dilution of step b) and reaching the conclusion of step c) is 2 hours or less, preferably 1 hour 30 minutes or less, and more preferably one hour or less.

10. A determination method according to any one of claims 1 to 9, **characterized in that** the infectious agent is a bacterium, a virus, a fungus, or a parasite.
